(19) Europäisches Patentamt / European Patent Office / Office européen des brevets

(11) **EP 4 239 324 B1**

(12) **EUROPEAN PATENT SPECIFICATION**

(45) Date of publication and mention
of the grant of the patent:
**15.01.2025 Bulletin 2025/03**

(51) International Patent Classification (IPC):
***G01N 23/201*** *(2018.01)*     ***G01N 33/44*** *(2006.01)*
***G01N 23/083*** *(2018.01)*     *G01N 15/08* *(2006.01)*

(21) Application number: **23154364.6**

(22) Date of filing: **01.02.2023**

(52) Cooperative Patent Classification (CPC):
**G01N 23/201; G01N 15/088; G01N 23/083;
G01N 33/445;** G01N 2015/0846; G01N 2223/649

(54) **METHOD FOR DETERMINING THE VOID VOLUME FRACTION IN RUBBER**

VERFAHREN ZUR BESTIMMUNG DES PORENVOLUMENANTEILS IN GUMMI

PROCÉDÉ DE DÉTERMINATION DE LA FRACTION VOLUMIQUE DE VIDE DANS LE CAOUTCHOUC

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **02.03.2022 JP 2022032075**

(43) Date of publication of application:
**06.09.2023 Bulletin 2023/36**

(73) Proprietor: **Sumitomo Rubber Industries, Ltd.
Kobe-shi, Hyogo 651-0072 (JP)**

(72) Inventor: **SHIOZAWA, Tomomi
Kobe-shi, Hyogo, 651-0072 (JP)**

(74) Representative: **Manitz Finsterwald
Patent- und Rechtsanwaltspartnerschaft mbB
Martin-Greif-Strasse 1
80336 München (DE)**

(56) References cited:
- **YOUNG R. J. ET AL: "Characterization of filled rubbers using small-angle X-ray scattering", J MATER SCI, vol. 21, April 1986 (1986-04-01), pages 1211 - 1218, XP093062105, DOI: 10.1007/BF00553253**
- **KANEKO S. ET AL: "Void formation in a filled SBR rubber determined by small-angle X-ray scattering", JOURNAL OF APPLIED POLYMER SCIENCE, vol. 26, no. 12, December 1981 (1981-12-01), US, pages 4175 - 4192, XP093062099, ISSN: 0021-8995, DOI: 10.1002/app.1981.070261219**
- **BLUME ANKE ET AL: "Void Volume Measurement - an alternative Approach to Determine the Initial Structure of Silica", KGK KAUTSCHUK, GUMMI, KUNSTSTOFFE, vol. 71, June 2018 (2018-06-01), pages 70 - 74, XP093062109**

**Description**

TECHNICAL FIELD

The present invention relates to a method of evaluating a rubber material.

BACKGROUND ART

It is known that small voids (cavities) may be formed in a rubber material when strain is applied thereto (see, for example, Non-Patent Literatures 1 and 2). YOUNG R. J. ET AL: "Characterization of filled rubbers using small-angle X-ray scattering", J MATER SCI, vol. 21, April 1986, pages 1211-1218, XP093062105, DOI: 10.1007/BF00553253 discloses a method for the determination of voids in a rubber using SAXS .

CITATION LIST

NON PATENT LITERATURE

Non-Patent Literature 1: Macromolecules 2012, 45, 1529-1543
Non-Patent Literature 2: Macromolecules 2013, 46, 900-913

SUMMARY OF INVENTION

TECHNICAL PROBLEM

In Non-Patent Literatures 1 and 2, the change in scattering intensity of a rubber material that occurs with void formation is measured by small-angle X-ray scattering analysis (SAXS analysis) to quantitatively evaluate voids. Such methods require measurements over a wide region of wavenumbers. In practice, however, since the area of the view plane of the detector is limited, the wavenumber region to be measured is limited. Thus, there is still room for improvement in that voids within some region cannot be observed.
The present invention aims to solve the aforementioned problem and provide an evaluation method that can easily evaluate the percentage of voids in a rubber material.

SOLUTION TO PROBLEM

The present invention relates to an evaluation method, comprising measuring a transmittance and thickness of a rubber material with no strain applied thereto and measuring a transmittance of the rubber material with the strain applied thereto and measuring or calculating from the following Equation (3) a thickness of the rubber material with the strain applied thereto, and evaluating a percentage of voids in the rubber material with a strain applied thereto based on a $\varphi_{void}$ calculated from the following Equation (1) using said transmittances and thicknesses, ,

$$\emptyset_{void} = 1 - \rho_s/\rho_0 = 1 - \ln\left(\frac{I_s}{I_0}\right)/\ln\left(\frac{I}{I_0}\right) \times (t_0/t_s) \qquad (1)$$

wherein

$\rho_0$: a density of the rubber material with no strain applied thereto,
$\rho_s$: a density of the rubber material with the strain applied thereto,
$I/I_0$: the transmittance of the rubber material with no strain applied thereto,
$I_s/I_0$: the transmittance of the rubber material with the strain applied thereto,
$I_0$: an intensity of a beam incident on the rubber material,
$I$: an intensity of the beam transmitted through the rubber material with no strain applied thereto,
$I_s$: an intensity of the beam transmitted through the rubber material with the strain applied thereto,
$t_0$: the thickness of the rubber material with no strain applied thereto, and
$t_s$: the thickness of the rubber material with the strain applied thereto,

$$t_s = t_0 / \sqrt{\epsilon/100} \qquad (3)$$

wherein

$t_0$: the thickness of the rubber material with no strain applied thereto,
$t_s$: the thickness of the rubber material with the strain applied thereto, and
$\epsilon$: the amount (%) of strain.

ADVANTAGEOUS EFFECTS OF INVENTION

According to the present invention, the percentage of voids in a rubber material with a strain applied thereto can be easily evaluated based on the $\varphi_{void}$ calculated from Equation (1) using the transmittance and thickness of the rubber material with no strain applied thereto and the transmittance and thickness of the rubber material with the strain applied thereto.

DESCRIPTION OF EMBODIMENTS

The present invention relates to an evaluation method measuring a transmittance and thickness of a rubber material with no strain applied thereto and measuring a transmittance of the rubber material with the strain applied thereto and measuring or calculating from the following Equation (3) a thickness of the rubber material with the strain applied thereto, and evaluating a percentage of voids in the rubber material with a strain applied thereto based on a $\varphi_{void}$ calculated from the following Equation (1) using said transmittances and thicknesses,

$$\emptyset_{\mathbf{void}} = 1 - \rho_s / \rho_0 = 1 - \ln\left(\frac{I_s}{I_0}\right) / \ln\left(\frac{I}{I_0}\right) \times (t_0/t_s) \qquad (1)$$

wherein

$\rho_0$: the density of the rubber material with no strain applied thereto,
$\rho_s$: the density of the rubber material with the strain applied thereto,
$I/I_0$: the transmittance of the rubber material with no strain applied thereto,
$I_s/I_0$: the transmittance of the rubber material with the strain applied thereto,
$I_0$: the intensity of the beam incident on the rubber material,
$I$: the intensity of the beam transmitted through the rubber material with no strain applied thereto,
$I_s$: the intensity of the beam transmitted through the rubber material with the strain applied thereto,
$t_0$: the thickness of the rubber material with no strain applied thereto, and
$t_s$: the thickness of the rubber material with the strain applied thereto ,

$$t_s = t_0 / \sqrt{\epsilon/100} \qquad (3)$$

wherein

$t_0$: the thickness of the rubber material with no strain applied thereto,
$t_s$: the thickness of the rubber material with the strain applied thereto, and
$\epsilon$: the amount (%) of strain.

When voids are formed in a rubber material, the rubber material usually has an increased volume while maintaining the mass, resulting in a lower density ($\rho_s < \rho_0$). Thus, a rubber material having a higher percentage of voids has a larger $\varphi_{void}$ calculated by "1 - $\rho_s/\rho_0$". This relationship may be used to evaluate (quantitatively evaluate) the percentage of voids from $\varphi_{void}$. Moreover, since $\varphi_{void}$ can be calculated only from the transmittance and thickness of the rubber material, the percentage of voids can be evaluated easily. Furthermore, since according to the present invention, the voids present in the volume irradiated with X-rays are evaluable, the voids in the entire rubber material can be evaluated by irradiating the entire rubber material with X-rays.
Here, the transmittance ($I/I_0$) of the material is generally determined by the formulation of the material and represented by Equation (2) below. Thus, "1 - $\rho_s/\rho_0$" may be transformed into an equation consisting of transmittance and thickness using Equation (2):

$$\frac{I}{I_0} = \exp(-\mu t) = \exp(-\mu_{\mathrm{m}}\rho t) \qquad (2)$$

wherein

I: the intensity of the beam incident on the material,
$I_0$: the intensity of the beam transmitted through the material,
$\mu$: the linear absorption coefficient,
$\mu_{\mathrm{m}}$: the mass absorption coefficient,
$\rho$: the density of the material, and
t: the thickness of the material.

The thickness ($t_s$) of the rubber material with the strain applied thereto in Equation (1) may be determined by measuring the actual thickness, or may be calculated from the following Equation (3):

$$t_s = t_0/\sqrt{\in/100} \qquad (3)$$

wherein

$t_0$: the thickness of the rubber material with no strain appli ed thereto,
$t_s$: the thickness of the rubber material with the strain applied thereto, and
$\varepsilon$: the amount (%) of strain.

Assuming that the rubber material deforms at a Poisson's ratio of 0.5, since the volume does not change, the rubber material can be regarded to deform at the same rate in the direction orthogonal to the strain. Thus, the thickness ($t_s$) of the rubber material with the strain applied thereto can be calculated from Equation (3). The use of Equation (3) makes it possible to more easily evaluate the percentage of voids in the rubber material.
Here, the voids of all sizes that occupy the volume can be evaluated based on $\varphi_{void}$. There is no lower limit of the evaluable size, while the upper limit thereof is the volume irradiated with X-rays.
Examples of the beam used to irradiate the rubber material include X-rays and light, with X-rays being preferred.
When X-rays are used as the beam, the transmittance and scattering intensity of the rubber material may be measured by SAXS analysis. The scattering angle for the SAXS analysis is usually not more than 10 degrees.
The SAXS analysis is usually performed in a region of q represented by the following equation:

$$q = \frac{4\pi \sin(\theta/2)}{\lambda}$$

wherein

$\theta$: scattering angle; and
$\lambda$: wavelength of X-rays or neutrons.

The region of q preferably includes 0.001 Å$^{-1}$ < q < 0.05 Å$^{-1}$.
The X-rays scattered in the SAXS analysis may be detected by an X-ray detector, and the X-ray detection data from the X-ray detector may be used to generate an image using an image processor or the like.
Examples of the X-ray detector include two-dimensional detectors such as X-ray films, nuclear emulsion plates, X-ray image pickup tubes, X-ray fluorescent amplifiers, X-ray image intensifiers, X-ray imaging plates, X-ray CCDs, and X-ray amorphous materials; and line sensor one-dimensional detectors. The X-ray detector may be selected appropriately depending on the type or conditions of the polymer material to be analyzed, or other factors.
The image processor may appropriately be a common one that can generate X-ray scattering images based on the X-ray detection data from the X-ray detector.
The strain to be applied to the rubber material is preferably an elongational strain, more preferably a uniaxial elongational strain. For example, a uniaxial elongational strain may be applied, for example, by holding the rubber material between a pair of opposing jigs and then elongating the rubber material with the jigs in the respective opposing directions, or by holding the rubber material between a pair of opposing jigs and then elongating the rubber material with one of the jigs, with the other jig being fixed.

The elongation rate at which the elongational strain is applied to the rubber material is usually 100 mm/min to 500 mm/min.

Although the rubber material may have any shape, it preferably has a plate shape or a dumbbell shape as set forth in JIS K 6251 because such a shape facilitates the application of a uniform elongational strain.

The thickness of the rubber material with no strain applied thereto is usually 1 mm to 2 mm.

Examples of rubber components that may be contained in the rubber material include diene rubbers such as isoprene-based rubbers, styrene-butadiene rubbers (SBR), polybutadiene rubbers (BR), acrylonitrile-butadiene rubbers (NBR), chloroprene rubbers (CR), butyl rubbers (IIR), and styrene-isoprene-butadiene copolymer rubbers (SIBR). Each of these rubbers may be used alone, or two or more of these may be used in combination.

The rubber material may contain a filler. Examples of the filler include silica, carbon black, calcium carbonate, talc, alumina, clay, aluminum hydroxide, aluminum oxide, and mica. Preferred among these is silica or carbon black.

The rubber material may contain additives such as stearic acid, zinc oxide, sulfur, and vulcanization accelerators, in addition to the rubber components and fillers.

The rubber material can be prepared by a usual method. Specifically, for example, the rubber material may be prepared by kneading the compounding materials using a kneading machine such as a Banbury mixer or an open roll mill, and then vulcanizing the kneaded mixture.

EXAMPLES

The present invention will be specifically described with reference to examples. The examples are not intended to limit the scope of the present invention.

<Formulation of rubber material>

Styrene-butadiene rubber (HPR850 available from JSR Corporation): 100 parts
Silica (VN3 available from Evonik Degussa): 53 parts
Silane coupling agent: Si266 (bis(3-triethoxysilyl-propyl)disulfide) available from Evonik Degussa): 4.26 parts
Sulfur (powdered sulfur available from Tsurumi Chemical Industry Co., Ltd.): 1.5 parts
Vulcanization accelerator 1 (NOCCELER NS (N-tert-butyl-2-benzothiazylsulfenamide) available from Ouchi Shinko Chemical Industrial Co., Ltd.): 2.0 parts
Vulcanization accelerator 2 (NOCCELER D (1,3-diphenylguanidine) available from Ouchi Shinko Chemical Industrial Co., Ltd.): 2.3 parts

<Method of preparing rubber material>

Following the formulation recipe, the compounding components other than the sulfur and vulcanization accelerators were kneaded in a 1.77 L internal Banbury mixer for 3 to 5 minutes until the temperature reached 150°C, to obtain a base-kneaded rubber compound. Next, the base-kneaded rubber compound was kneaded with the sulfur and vulcanization accelerators in an open roll mill, and the resulting kneaded mixture was vulcanized to obtain a rubber material.

The rubber material was sliced into a thickness of 1 mm and then punched into a dumbbell shape as set forth in JIS K 6251 to prepare a specimen, which was used in the following measurements.

<Example>

(SAXS analysis)

The experiments were conducted at BL20XU of SPring-8. Ion chambers were disposed in front of and behind the specimen, and the specimen was irradiated with X-rays for an exposure time of one second at two-second intervals to determine the scattering intensity and transmittance.

Separately, the same procedure was followed while applying a uniaxial elongational strain to the specimen, to determine the scattering intensity and transmittance. The elongation rate of the specimen was 50 mm/min. The following describes the other conditions.

Brilliance of X-rays (8 keV): $9.5 \times 10^{15}$
photons/s/mrad$^2$/mm$^2$/0.1%bw
Number of photons of X-rays: $10^9$ to $10^{10}$ photons/s
Distance from specimen to detector: 2.58 m
Detector: PILATUS 100K (Dectris AG)

(Data processing)

Equation (1) was used to calculate the $\varphi_{void}$. Table 1 shows the results.
Here, the $t_s$ was calculated from the amount (%) of strain using Equation (3).

[Table 1]

| Amount (%) of strain | $\phi$ void |
|---|---|
| 0 | 0 |
| 100 | 0.019 |
| 150 | 0.053 |
| 200 | 0.065 |

Table 1 shows that in the example the $\varphi_{void}$ increases as the amount of strain increases. Thus, it was demonstrated that the quantitative evaluation of voids can be achieved by comparing the values of $\varphi_{void}$.

<Comparative Example>

The noise and background were subtracted from the scattering intensity measured in the example, and the $\varphi_{void}$ was calculated using equations (1) to (3) of Non-Patent Literature 2. The change in specimen thickness due to the elongation was corrected using the thickness obtained from the transmittance.
In the comparative example, since the observable wavenumber region was narrow, no quantitative data within the unobserved region was obtained.

**Claims**

1. An evaluation method, comprising
   measuring a transmittance and thickness of a rubber material with no strain applied thereto and measuring a transmittance of the rubber material with the strain applied thereto and measuring or calculating from the following Equation (3) a thickness of the rubber material with the strain applied thereto, and

   evaluating a percentage of voids in the rubber material with a strain applied thereto based on a $\varphi_{void}$ calculated from the following Equation (1) using said transmittances and thicknesses,

$$\emptyset_{void} = 1 - \rho_s/\rho_0 = 1 - \ln\left(\frac{I_s}{I_0}\right)/\ln\left(\frac{I}{I_0}\right) \times (t_0/t_s) \qquad (1)$$

   wherein
   $\rho_0$: a density of the rubber material with no strain applied thereto,
   $\rho_s$: a density of the rubber material with the strain applied thereto,
   $I/I_0$: the transmittance of the rubber material with no strain applied thereto,
   $I_s/I_0$: the transmittance of the rubber material with the strain applied thereto,
   $I_0$: an intensity of a beam incident on the rubber material, I: an intensity of the beam transmitted through the rubber material with no strain applied thereto,
   $I_s$: an intensity of the beam transmitted through the rubber material with the strain applied thereto,
   $t_0$: the thickness of the rubber material with no strain applied thereto, and
   $t_s$: the thickness of the rubber material with the strain applied thereto,

$$t_s = t_0/\sqrt{\in/100} \qquad (3)$$

wherein

$t_0$: the thickness of the rubber material with no strain applied thereto,
$t_s$: the thickness of the rubber material with the strain applied thereto, and
$\varepsilon$: the amount (%) of strain.

2. The evaluation method according to claim 1,
wherein the thickness of the rubber material with the strain applied thereto is calculated assuming that the rubber material deforms at a Poisson's ratio of 0.5.

3. The evaluation method according to claim 1 or 2,
wherein the strain is an elongational strain.

4. The evaluation method according to Claim 3,
wherein the elongational strain is a uniaxial elongational strain.

5. The evaluation method according to Claim 3 or 4,
wherein an elongation rate at which the elongational strain is applied is 100 mm/min to 500 mm/min.

6. The evaluation method according to any one of claims 1 to 5,
wherein the beam is X-rays.

7. The evaluation method according to claim 6,
wherein the transmittance and scattering intensity of the rubber material are measured by small-angle X-ray scattering analysis.

8. The evaluation method according to Claim 7,
wherein the small-angle X-ray scattering analysis is performed in a region of q represented by the following equation:

$$q = \frac{4\pi \sin(\theta/2)}{\lambda}$$

wherein

$\theta$: scattering angle; and
$\lambda$: wavelength of X-rays or neutrons.

9. The evaluation method according to Claim 8,
wherein the region of q includes 0.001 $\text{Å}^{-1}$ < q < 0.05 $\text{Å}^{-1}$.

10. The evaluation method according to any one of claims 1 to 9,
wherein the rubber material contains a filler.

11. The evaluation method according to Claim 10,
wherein the filler is at least one selected from the group consisting of silica, carbon black, calcium carbonate, talc, alumina, clay, aluminum hydroxide, aluminum oxide, and mica.

12. The evaluation method according to any combination with any one of Claims 1 to 11,
wherein the rubber material contains at least one rubber component selected from the group consisting of isoprene-based rubbers, styrene-butadiene rubbers, polybutadiene rubbers, acrylonitrile-butadiene rubbers, chloroprene rubbers, butyl rubbers, and styrene-isoprene-butadiene copolymer rubbers.

13. The evaluation method according to any combination with any one of Claims 1 to 12,
wherein a thickness of the rubber material with no strain applied thereto is 1 mm to 2 mm.

**Patentansprüche**

1. Bewertungsverfahren, umfassend
Messen eines Transmissionsgrades und einer Dicke eines Kautschukmaterials ohne daran angewandte Verformung und Messen eines Transmissionsgrades des Kautschukmaterials mit der daran angewandten Verformung und Messen oder aus der folgenden Gleichung (3) Berechnen einer Dicke der Kautschukmaterials mit der daran angewandten Verformung, und

   Bewerten eines prozentualen Anteils an Hohlräumen in dem Kautschukmaterial mit einer daran angewandten Verformung, basierend auf einem aus der folgenden Gleichung (1) unter Verwendung der Transmissionsgrade und Dicken berechneten $\varphi_{Hohlraum}$,

$$\emptyset_{Hohlraum} = 1 - \rho_s/\rho_0 = 1 - \ln\left(\frac{I_s}{I_0}\right)/\ln\left(\frac{I}{I_0}\right) \times (t_0/t_s) \qquad (1)$$

   wobei
   $\rho_0$: eine Dichte des Kautschukmaterials ohne daran angewandte Verformung,
   $\rho_s$: eine Dichte des Kautschukmaterials mit der daran angewandten Verformung,
   $I/I_0$: der Transmissionsgrad des Kautschukmaterials ohne daran angewandte Verformung,
   $I_s/I_0$: der Transmissionsgrad des Kautschukmaterials mit der daran angewandten Verformung,
   $I_0$: eine Intensität eines auf das Kautschukmaterial einfallenden Strahls,
   $I$: eine Intensität des durch das Kautschukmaterial ohne daran angewandte Verformung transmittierten Strahls,
   $I_s$: eine Intensität des durch das Kautschukmaterial mit der daran angewandten Verformung transmittierten Strahls,
   $t_0$: die Dicke des Kautschukmaterials ohne daran angewandte Verformung, und
   $t_s$: die Dicke des Kautschukmaterials mit der daran angewandten Verformung,

$$t_s = t_0/\sqrt{\epsilon/100} \qquad (3)$$

   wobei

   $t_0$: die Dicke des Kautschukmaterials ohne daran angewandte Verformung,
   $t_s$: die Dicke des Kautschukmaterials mit der daran angewandten Verformung, und
   $\epsilon$: das Maß (%) an Verformung.

2. Bewertungsverfahren nach Anspruch 1,
wobei die Dicke des Kautschukmaterials mit der daran angewandten Verformung unter der Annahme berechnet wird, dass sich das Kautschukmaterial mit einer Poissonzahl von 0,5 verformt.

3. Bewertungsverfahren nach Anspruch 1 oder 2,
wobei die Verformung eine dehnende Verformung ist.

4. Bewertungsverfahren nach Anspruch 3,
wobei die dehnende Verformung eine uniaxiale dehnende Verformung ist.

5. Bewertungsverfahren nach Anspruch 3 oder 4,
wobei eine Dehnungsrate, mit der die dehnende Verformung angewandt wird, 100 mm/min bis 500 mm/min beträgt.

6. Bewertungsverfahren nach einem der Ansprüche 1 bis 5, wobei der Strahl Röntgenstrahlen ist.

7. Bewertungsverfahren nach Anspruch 6,
wobei der Transmissionsgrad und die Streuintensität des Kautschukmaterials durch Kleinwinkelröntgenstreuungsanalyse gemessen werden.

8. Bewertungsverfahren nach Anspruch 7,
wobei die Kleinwinkelröntgenstreuungsanalyse in einem Bereich von q durchgeführt wird, dargestellt durch die folgende Gleichung:

$$q = \frac{4\pi \sin(\theta/2)}{\lambda}$$

wobei

$\theta$: Streuwinkel; und

$\lambda$: Wellenlänge von Röntgenstrahlen oder Neutronen.

**9.** Bewertungsverfahren nach Anspruch 8,
wobei der Bereich von q $0{,}001 \ \text{Å}^{-1} < q < 0{,}05 \ \text{Å}^{-1}$ umfasst.

**10.** Bewertungsverfahren nach einem der Ansprüche 1 bis 9,
wobei das Kautschukmaterial einen Füllstoff enthält.

**11.** Bewertungsverfahren nach Anspruch 10,
wobei der Füllstoff mindestens einer ausgewählt aus der Gruppe bestehend aus Siliziumdioxid, Ruß, Calciumcarbonat, Talk, Alumina, Ton, Aluminiumhydroxid, Aluminiumoxid und Glimmer ist.

**12.** Bewertungsverfahren nach einer Kombination mit einem der Ansprüche 1 bis 11,
wobei das Kautschukmaterial mindestens eine Kautschukkomponente ausgewählt aus der Gruppe bestehend aus Isoprenbasierten Kautschuken, Styrol-Butadien-Kautschuken, Polybutadien-Kautschuken, Acrylnitril-Butadien-Kautschuken, Chloropren-Kautschuken, Butylkautschuken und Styrol-Isopren-Butadien-Copolymer-Kautschuken enthält.

**13.** Bewertungsverfahren nach einer Kombination mit einem der Ansprüche 1 bis 12,
wobei eine Dicke des Kautschukmaterials ohne daran angewandte Verformung 1 mm bis 2 mm beträgt.

**Revendications**

**1.** Procédé d'évaluation, comprenant

la mesure d'un facteur de transmission et d'une épaisseur d'un matériau de caoutchouc sans contrainte appliquée à celui-ci et la mesure d'un facteur de transmission du matériau de caoutchouc avec la contrainte appliquée à celui-ci et la mesure ou le calcul à partir de l'équation (3) suivante d'une épaisseur du matériau de caoutchouc avec la contrainte appliquée à celui-ci et l'évaluation d'un pourcentage de cavités dans le matériau de caoutchouc avec une contrainte appliquée à celui-ci sur la base d'un $\varphi_{void}$ calculé à partir de l'équation (1) suivante en utilisant lesdits facteurs de transmission et épaisseurs,

$$\varphi_{void} = 1 - \rho_s/\rho_0 = 1 - \ln\left(\frac{I_s}{I_0}\right)/\ln\left(\frac{I}{I_0}\right) \times (t_0/t_s) \qquad (1)$$

$\rho_0$ : une densité du matériau de caoutchouc sans contrainte appliquée à celui-ci,
$\rho_s$ : une densité du matériau de caoutchouc avec la contrainte appliquée à celui-ci,
$I/I_0$ : le facteur de transmission du matériau de caoutchouc sans contrainte appliquée à celui-ci,
$I_s/I_0$ : le facteur de transmission du matériau de caoutchouc avec la contrainte appliquée à celui-ci,
$I_0$ : une intensité d'un faisceau incident sur le matériau de caoutchouc,
$I$ : une intensité du faisceau transmis à travers le matériau de caoutchouc sans contrainte appliquée à celui-ci,
$I_s$ : une intensité du faisceau transmis à travers le matériau de caoutchouc avec la contrainte appliquée à celui-ci,
$t_0$ : l'épaisseur du matériau de caoutchouc sans contrainte appliquée à celui-ci, et
$t_s$ : l'épaisseur du matériau de caoutchouc avec la contrainte appliquée à celui-ci,

$$t_s = t_0/\sqrt{\epsilon/100} \qquad (3)$$

où
$t_0$ : l'épaisseur du matériau de caoutchouc sans contrainte appliquée à celui-ci,

$t_s$ : l'épaisseur du matériau de caoutchouc avec la contrainte appliquée à celui-ci, et

$\varepsilon$ : la quantité (%) de contrainte.

2. Procédé d'évaluation selon la revendication 1,
dans lequel l'épaisseur du matériau de caoutchouc avec la contrainte appliquée à celui-ci est calculée en supposant que le matériau de caoutchouc se déforme à un rapport de Poisson de 0,5.

3. Procédé d'évaluation selon la revendication 1 ou 2,
dans lequel la contrainte est une contrainte d'élongation.

4. Procédé d'évaluation selon la revendication 3,
dans lequel la contrainte d'élongation est une contrainte d'élongation uniaxiale.

5. Procédé d'évaluation selon la revendication 3 ou 4,
dans lequel une vitesse d'élongation à laquelle la contrainte d'élongation est appliquée est de 100 mm/min à 500 mm/min.

6. Procédé d'évaluation selon l'une quelconque des revendications 1 à 5, dans lequel le faisceau est les rayons X.

7. Procédé d'évaluation selon la revendication 6,
dans lequel le facteur de transmission et l'intensité de diffusion du matériau de caoutchouc sont mesurés par analyse de diffusion de rayons X à petit angle.

8. Procédé d'évaluation selon la revendication 7,

dans lequel l'analyse de diffusion de rayons X à petit angle est effectuée dans une région de q représentée par l'équation suivante :

$$q = \frac{4\pi \sin(\theta/2)}{\lambda}$$

où
$\theta$ : angle de diffusion ; et
$\lambda$ : longueur d'onde des rayons X ou des neutrons.

9. Procédé d'évaluation selon la revendication 8,
dans lequel la région de q comprend $0,001 \text{ Å}^{-1} < q < 0,05 \text{ Å}^{-1}$.

10. Procédé d'évaluation selon l'une quelconque des revendications 1 à 9, dans lequel le matériau de caoutchouc contient une charge.

11. Procédé d'évaluation selon la revendication 10,
dans lequel la charge est au moins un choisi dans le groupe constitué par la silice, le noir de charbon, le carbonate de calcium, le talc, l'alumine, l'argile, l'hydroxyde d'aluminium, l'oxyde d'aluminium, et le mica.

12. Procédé d'évaluation selon une quelconque combinaison avec l'une quelconque des revendications 1 à 11,
dans lequel le matériau de caoutchouc contient au moins un composant de caoutchouc choisi dans le groupe constitué par les caoutchoucs à base d'isoprène, les caoutchoucs de styrène-butadiène, les caoutchoucs de polybutadiène, les caoutchoucs d'acrylonitrile-butadiène, les caoutchoucs de chloroprène, les caoutchoucs de butyle, et les caoutchoucs de copolymère styrène-isoprène-butadiène.

13. Procédé d'évaluation selon une quelconque combinaison avec l'une quelconque des revendications 1 à 12,
dans lequel une épaisseur du matériau de caoutchouc sans contrainte appliquée à celui-ci est de 1 mm à 2 mm.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YOUNG R. J. et al.** Characterization of filled rubbers using small-angle X-ray scattering. *J MATER SCI*, April 1986, vol. 21, 1211-1218

- *Macromolecules*, 2012, vol. 45, 1529-1543
- *Macromolecules*, 2013, vol. 46, 900-913